# FASCICULE DE BREVET EUROPEEN

(11) **EP 3 988 146 B1**
(45) Date de publication et mention de la délivrance du brevet: **08.04.2026**
(21) Numéro de dépôt: 21203976.2
(22) Date de dépôt: 21.10.2021
(51) Int. Cl.: A61M 5/178, A61J 1/20, A61M 5/31, A61M 5/165

(54) **EMBOUT DE FILTRATION D'UN PRODUIT INJECTABLE ET KIT DE FILTRATION ASSOCIÉ**
FILTRATIONSENDSTÜCK FÜR EIN INJIZIERBARES PRODUKT UND DAS ZUGEHÖRIGE FILTRATIONSKIT
FILTRATION TIP FOR AN INJECTABLE PRODUCT AND ASSOCIATED FILTRATION KIT

(30) Priorité: 22.10.2020 FR 2010848
(43) Date de publication de la demande: 27.04.2022
(73) Titulaire: EDEC - LABORATOIRE CAT, 45260 Montereau (FR)
(72) Inventeur: BAUDRY, Nicolas, 60320 SAINT-SAUVEUR (FR)
(74) Mandataire: Lavoix

(56) Documents cités:
- FR-A1- 2 850 564
- FR-A1- 2 924 019
- FR-A1- 3 034 995
- FR-A1- 3 083 444

## Description

La présente invention concerne un embout de filtration d'un produit injectable destiné à être monté à l'extrémité distale d'une seringue.

Il est connu, notamment de FR2924019A1 et de FR3083444A1, d'utiliser un embout de filtration monté à l'extrémité distale d'une seringue dans le but de filtrer les impuretés présentes dans un produit destiné à être injecté par la seringue.

Un tel embout de filtration comprend un corps d'embout définissant une lumière axiale de passage du produit injectable. Le corps d'embout comprend une collerette, une partie intermédiaire destinée à recevoir une extrémité distale de la seringue et une tête plate d'aspiration du produit injectable définissant une ouverture d'aspiration du produit injectable sur laquelle est montée une membrane filtrante.

La partie intermédiaire d'un tel embout comporte intérieurement plusieurs parties tronconiques raccordées les unes aux autres par un épaulement ou par un raccord tronconique. Chaque partie tronconique est adaptée pour recevoir un type particulier de seringue ayant une forme et un diamètre prédéfinis.

Un tel embout ne donne pas entière satisfaction. Il présente une structure compliquée qui peut être difficile et coûteuse à fabriquer. Par ailleurs, il ne s'adapte qu'à des types bien définis de seringues.

Un but de l'invention est de proposer un embout de filtration simple et économique apte à être monté de manière étanche sur des seringues de formes et de diamètres variés.

A cet effet, l'invention a pour objet un embout de filtration selon la revendication 1.

L'embout de filtration d'un produit injectable selon l'invention peut comprendre l'une ou plusieurs caractéristiques des revendications 2 à 8, prise(s) isolement ou suivant toute combinaison techniquement possible.

L'invention a également pour objet un kit de filtration selon la revendication 9.

Le kit de filtration d'un produit injectable selon l'invention peut comprendre l'une ou plusieurs caractéristiques des revendications 10 et 11, prise(s) isolement ou suivant toute combinaison techniquement possible.

L'invention a également pour objet un ensemble de kits de filtration selon la revendication 12.

L'invention sera mieux comprise à la lecture de la description qui va suivre, donnée uniquement à titre d'exemple, et faite en se référant aux dessins annexés, sur lesquels :
- [Fig 1] la figure 1 est une vue en perspective d'un embout de filtration selon l'invention.
- [Fig 2] la figure 2 est une vue en coupe verticale de l'embout de filtration de la figure 1.
- [Fig 3] la figure 3 est une vue de dessous de l'embout de filtration de la figure 1.
- [Fig 4] la figure 4 est une vue en coupe verticale de l'embout de filtration de la figure 1 monté sur l'extrémité distale d'une première seringue.
- [Fig 5] la figure 5 est une vue en coupe verticale de l'embout de filtration de la figure 1 monté sur l'extrémité distale d'une deuxième seringue.
- [Fig 6] la figure 6 est une vue en coupe verticale de l'embout de filtration de la figure 1 monté sur l'extrémité distale d'une troisième seringue.
- [Fig 7] la figure 7 est une vue en coupe verticale de l'embout de filtration de la figure 1 monté sur l'extrémité distale d'une quatrième seringue.

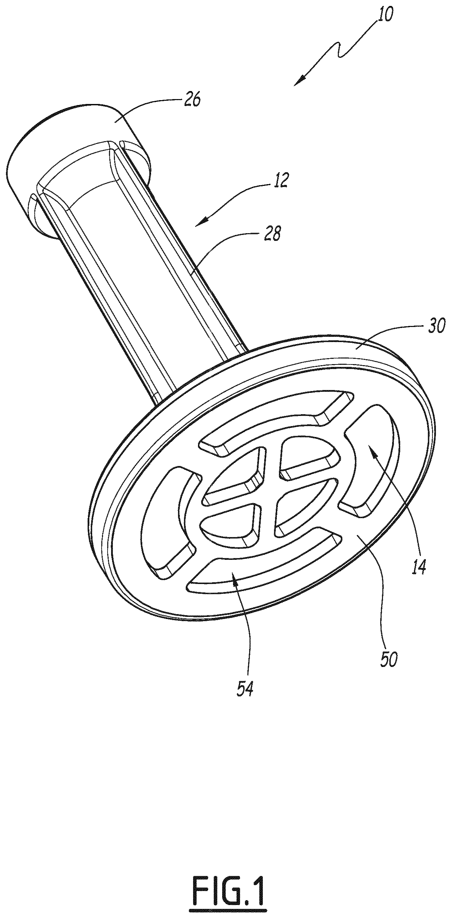

La figure 1 illustre un embout de filtration 10 d'un produit injectable comprenant un corps 12 d'embout et une membrane 14 filtrante.

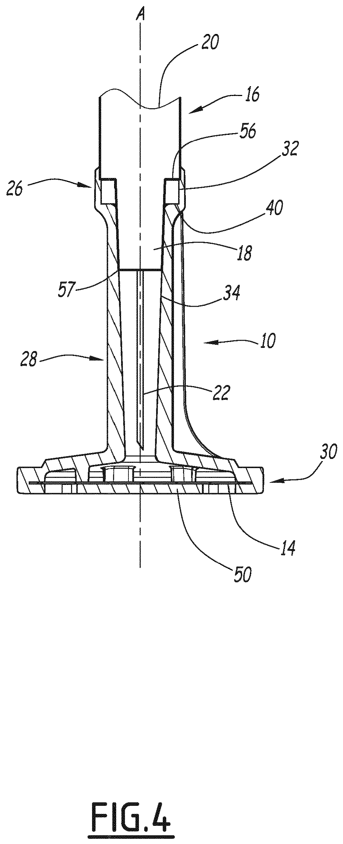

Comme illustré sur les figures 4 à 7, l'embout de filtration 10 est destiné à être monté sur une seringue 16, et plus particulièrement sur une extrémité distale 18 de la seringue 16.

La seringue 16 est, par exemple, une seringue d'injection d'un type classique s'étendant selon un axe longitudinal et définissant une extrémité distale 18 et un corps proximal 20. La seringue 16 d'injection comprend, avantageusement, à son extrémité distale 18, une aiguille 22 d'injection qui s'étend selon l'axe longitudinal de la seringue. L'aiguille d'injection 22 est fixe, par exemple sertie, ou est amovible.

Le produit injectable est, par exemple, un produit pharmaceutique contenant un principe actif et est, avantageusement, sous forme liquide. Le produit injectable est destiné à être injecté par le biais de la seringue 16.

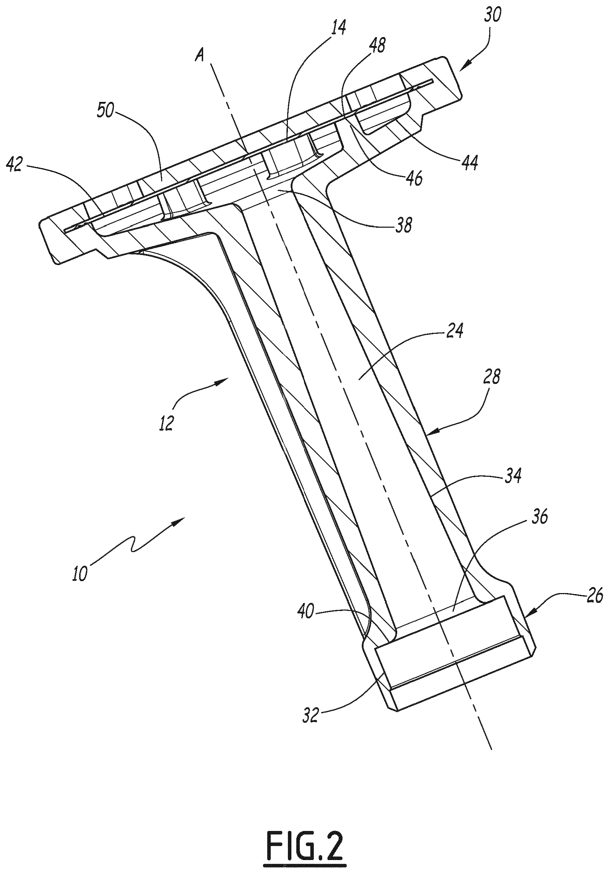

Comme illustré sur la figure 2, le corps d'embout 12 est de forme creuse et définit une lumière 24 axiale de passage du produit injectable dans l'embout 10.

La lumière axiale 24 s'étend selon un axe A. Lorsque l'embout de filtration 10 est monté sur une seringue 16 comme illustré sur les figures 4 à 7, l'axe A coïncide avec l'axe longitudinal de la seringue 16.

Le corps d'embout 12 comprend une collerette 26, une partie intermédiaire 28 et une tête plate 30 d'aspiration.

La collerette 26 définit une extrémité proximale du corps d'embout 12.

Comme illustré sur la figure 2, la collerette 26 définit une surface interne 32 cylindrique. Avantageusement, la surface interne 32 cylindrique s'étend selon l'axe A de la lumière 24.

La surface interne 32 cylindrique de la collerette 26 a, par exemple, un diamètre compris entre 5 mm et 12 mm et une hauteur comprise entre 2 mm et 4 mm.

La partie intermédiaire 28 du corps 12 d'embout est destinée à recevoir l'extrémité distale 18 de la seringue 16, et éventuellement, l'aiguille d'injection 22 de la seringue 16.

Comme illustré sur la figure 2, la partie intermédiaire 28 du corps d'embout 12 définit une surface interne 34 tronconique.

La surface interne tronconique 34 s'étend selon l'axe A de la lumière 24.

Avantageusement, la surface interne tronconique 34 délimite la périphérie de la lumière axiale 24.

La surface interne tronconique 34 s'étend depuis la collerette 26 jusqu'à la tête plate d'aspiration 30 de manière continue, et avantageusement sans aucun changement de pente. La surface interne 34 est plus particulièrement continument dérivable, depuis la collerette 26 jusqu'à la tête plate 30 d'aspiration.

L'angle au sommet de la surface interne 34 tronconique de la partie intermédiaire 28 est compris entre 2° et 10°.

La surface interne 34 converge donc de haut en bas. La dimension transversale de la surface interne 34 au niveau de l'interface 36 entre la collerette 26 et la partie intermédiaire 28 est supérieure à la dimension transversale de la surface interne 34 au niveau de l'interface 38 entre la partie intermédiaire 28 et la tête plate 30 d'aspiration.

Avantageusement, l'interface 36 entre la collerette 26 et la partie intermédiaire 28 est de section transversale circulaire.

Comme cela est représenté sur la figure 2, le diamètre de la surface interne tronconique 34 au niveau de l'interface 36 entre la collerette 26 et la partie intermédiaire 28 est, par exemple, inférieur au diamètre de la surface interne cylindrique 32 de la collerette 26, de sorte à définir un épaulement 40 entre la collerette 26 et la partie intermédiaire 28.

La dimension transversale de la surface interne tronconique 34 au niveau de l'interface 38 entre la partie intermédiaire 28 et la tête plate 30 d'aspiration est par exemple comprise entre 1 mm et 5 mm.

Avantageusement, l'interface 38 entre la partie intermédiaire 28 et la tête plate 30 d'aspiration est de section transversale circulaire.

Le surface interne tronconique 34 a une hauteur, correspondant à la distance entre l'interface 36 avec la collerette 26 et l'interface 38 avec la tête plate d'aspiration 30, comprise entre 15 mm et 30 mm.

La tête plate d'aspiration 30 définit une extrémité distale du corps d'embout 12.

Comme illustré sur la figure 2, la tête plate 30 d'aspiration définit une cavité interne débouchant par une ouverture 42 d'aspiration du produit injectable.

L'ouverture d'aspiration 42 se situe distalement par rapport à l'interface 38 avec la partie intermédiaire 28. La tête plate d'aspiration 30 définit intérieurement une surface interne 44 tronconique inversée entre l'interface 38 avec la partie intermédiaire 28 et l'ouverture 42 d'aspiration qui délimite vers le haut la cavité interne.

L'angle au sommet de la surface interne 44 tronconique inversée est, par exemple, compris entre 60° et 80°.

La tête plate 30 d'aspiration présente une hauteur, par exemple, comprise entre 1 mm et 3 mm.

La dimension transversale de l'ouverture d'aspiration 42 est supérieure à la dimension transversale minimale de la lumière axiale 24.

Plus particulièrement, la dimension transversale de l'ouverture d'aspiration 42 est supérieure d'un rapport de plus de 10 à la dimension transversale de la surface interne tronconique 34 au niveau de l'interface 38 entre la tête plate 30 d'aspiration et la partie intermédiaire 28.

L'ouverture d'aspiration 42 est, par exemple, de forme circulaire de diamètre compris entre 10 mm et 30 mm.

Comme cela est visible sur la figure 2, la tête plate d'aspiration 30 comprend avantageusement des butées 46, qui font saillie dans la cavité interne.

Chaque butée 46 s'étend selon une direction sensiblement parallèle à l'axe A à l'intérieur de la surface interne tronconique inversée 44 et définit une extrémité libre 48 de butée.

Le corps d'embout 12 est en matière plastique, et plus particulièrement en matière plastique spécifique pour les applications médicales.

Le corps d'embout 12 est, par exemple, fabriqué d'une seule pièce par injection plastique dans un moule.

Comme cela est représenté sur la figure 2, la membrane filtrante 14 est montée dans l'ouverture d'aspiration 42 de la tête plate d'aspiration 30.

Plus particulièrement, la membrane filtrante 14 est disposée en appui sur l'extrémité libre 48 de chaque butée 46 de la tête plate d'aspiration 30.

La membrane filtrante 14 est apte à filtrer la composition du produit injectable, et notamment de filtrer les impuretés présentes dans le produit injectable. La membrane filtrante 14 présente, par exemple, une porosité comprise entre 0,05 µm et 0,25 µm, plus particulièrement entre 0,10 µm et 0,22 µm et avantageusement égale à 0,20 µm.

Avantageusement, la membrane filtrante 14 recouvre l'intégralité de l'ouverture 42 d'aspiration, de sorte que l'intégralité du produit injectable entrant dans l'embout 10 à travers l'ouverture d'aspiration 42 passe nécessairement à travers la membrane filtrante 14.

La membrane filtrante 14 est réalisable en polyamide, en polypropylène, en polyethersulfone ou en toute autre matière plastique appropriée.

La membrane filtrante 14 est avantageusement fine, et présente une épaisseur inférieure à 0,2 mm.

Avantageusement, comme illustré sur la figure 1, l'embout 10 comprend également une grille de maintien 50 de la membrane filtrante 14.

La grille de maintien 50 est située distalement par rapport à la membrane filtrante 14.

La membrane filtrante 14 est maintenue fixe en position entre l'extrémité libre 48 des butées 46 et la grille de maintien 50.

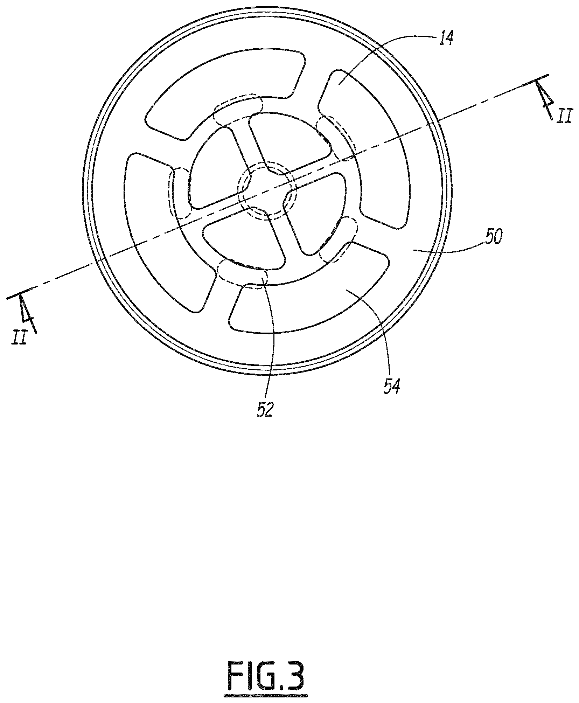

Comme illustré sur la figure 3, chaque butée 46 définit ainsi une zone de maintien 52 de la membrane filtrante 14 contre la grille de maintien 50.

Un tel maintien de la membrane filtrante 14 en plusieurs zones de maintien 52 évite que la membrane filtrante ne fléchisse et ne se perce en cas d'aspiration trop prononcée, tout en maintenant une surface de filtration élevée.

La grille de maintien 50 comprend des passages définissant des surfaces 54 de filtration du produit injectable.

L'ensemble des surfaces 54 de filtration définissent la surface utile sur laquelle le produit injectable va pouvoir rentrer en contact avec la membrane pour être filtré.

Avantageusement, la surface utile des surfaces 54 de filtration reste identique indépendamment de l'orientation de la grille de maintien 50.

La surface utile définit par les surfaces 54 de filtration est par exemple comprise entre 100 mm² et 200 mm².

La grille de maintien 50 est, par exemple, surmoulée ou clipsée sur la tête plate 30 d'aspiration, ou selon tout autre procédé de fabrication adapté.

Avantageusement, la grille de maintien 50 a une épaisseur comprise entre 0,5 mm et 1 mm. Un telle finesse de la grille de maintien 50 permet de réduire la perte de produit injectable non aspiré.

L'embout de filtration 10 est propre à recevoir des seringues 16 de structures très différentes comme cela va être décrit ci-dessous.

Par exemple, l'embout de filtration 10 est propre à être associé à une seringue 16 pour former un kit de filtration.

Le kit de filtration comprend un embout de filtration 10, comme décrit précédemment, et au moins une seringue 16.

Le kit de filtration comprend, avantageusement une boîte, une pochette ou tout autre contenant apte à contenir un embout de filtration 10 et au moins une seringue 16.

L'embout de filtration 10 est, avantageusement, disposé dans une poche fermée hermétiquement de manière stérile.

L'embout de filtration 10 est, par exemple, à usage unique.

La seringue 16 propre à être montée dans l'embout 10 peut présenter des extrémités distales 18 de forme ou/et de dimension transversale différentes.

Par exemple, comme cela est représenté sur la figure 4, la seringue 16 comprend une extrémité distale 18 de forme cylindrique raccordée à un corps proximal 20 de la seringue 16 par un épaulement 56 perpendiculaire à l'axe de la seringue 16.

L'extrémité distale 18 de la seringue 16 représentée sur la figure 4 est par exemple, de forme cylindrique s'étendant selon l'axe A et de section circulaire de diamètre compris entre 2 et 10 mm.

Le corps proximal 20 est également de forme cylindrique s'étendant selon l'axe A. Il présente un diamètre supérieur au diamètre de l'extrémité distale 18 et avantageusement de diamètre inférieur à la dimension transversale de la surface interne cylindrique 32 de la collerette 26.

L'épaulement 56 est entre l'extrémité distale 18 et le corps proximal 20. Comme indiqué plus haut, il est sensiblement perpendiculaire à l'axe A.

Comme cela est représenté sur la figure 4, l'extrémité distale 18 de la seringue 16 est propre à se monter dans l'embout 10, de sorte que l'extrémité distale 18 soit en contact avec la surface interne tronconique 34 de la partie intermédiaire 28 suivant une surface de de contact annulaire 57.

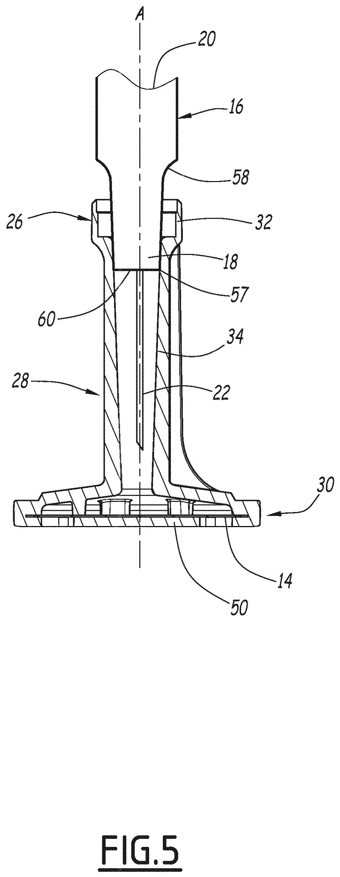

En variante, comme cela est représenté sur la figure 5, la seringue 16 comprend un extrémité distale 18 tronconique raccordée au corps proximal 20 de la seringue 16 par un épaulement 58 de forme concave arrondie.

L'extrémité distale 18 de la seringue 16 de forme tronconique représentée sur la figure 5 s'étend par exemple selon l'axe A avec un angle au sommet compris entre 3° et 5° entre l'épaulement 58 et un bord libre 60.

La dimension transversale du bord libre 60 de l'extrémité distale 18 est comprise entre 2 et 10 mm.

L'extrémité distale 18 de la seringue 16 illustrée sur la figure 5 est propre à se monter dans l'embout 10, de sorte que le bord libre 60 de l'extrémité distale 18 soit en contact avec la surface interne 34 tronconique de la partie intermédiaire 28 suivant une surface de contact annulaire 57.

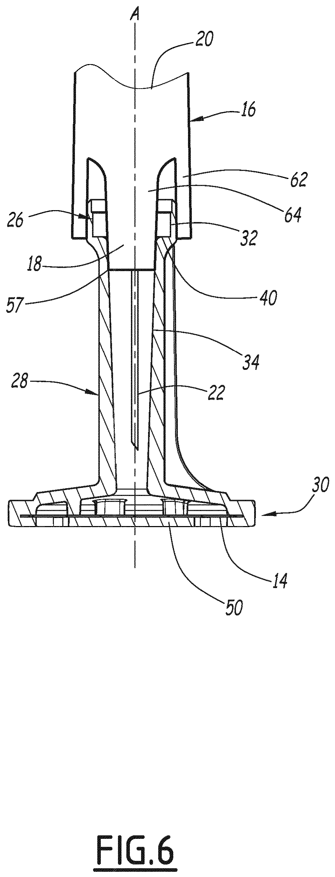

En variante, comme cela est représenté sur la figure 6, la seringue 16 comprend une jupe externe 62 autour de l'extrémité distale 18. Un espace annulaire 64 est défini entre la jupe externe 62 et l'extrémité distale 18 de la seringue 16.

Le diamètre de l'espace annulaire 64 est, par exemple, compris entre 2 et 10 mm.

La jupe externe 62 a un diamètre intérieur de jupe supérieur à la dimension transversale externe de la collerette 26 de l'embout 16.

L'extrémité distale 18 de la seringue 16 illustrée sur la figure 6 est propre à se monter dans l'embout 10, de sorte que l'extrémité distale 18 soit en contact avec la surface interne 34 tronconique de la partie intermédiaire 28 suivant une surface de contact annulaire 57. Lorsque l'embout 10 est monté sur la seringue 16, la périphérie interne de la jupe externe 62 de la seringue 16 est de préférence en contact extérieur avec la surface périphérique extérieure de la collerette 26 de l'embout 10.

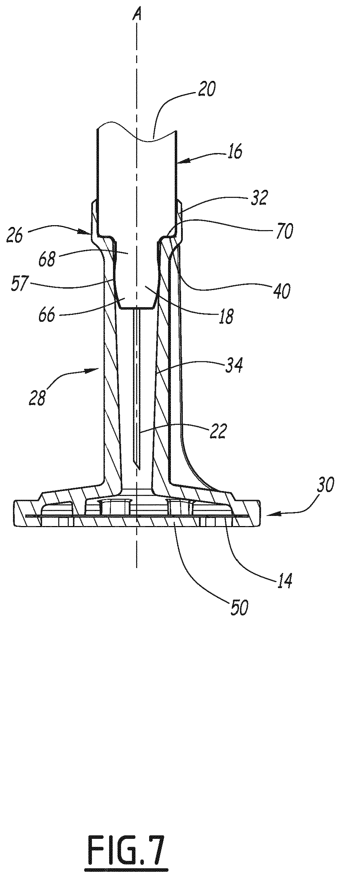

En variante, comme cela est représenté sur la figure 7, la seringue 16 comprend une extrémité distale 18 comprenant une partie distale 66 tronconique et une partie proximale 68 cylindrique raccordée au corps proximal 20 de la seringue 16 par un épaulement 70 perpendiculaire.

La partie distale 66 tronconique s'étend selon l'axe A d'angle au sommet compris entre 3° et 5°.

La partie proximale 68 est, par exemple, de diamètre compris entre 2 et 10 mm, et avantageusement inférieure à la dimension transversale maximale la surface interne 34 tronconique de la partie intermédiaire 28.

Le corps proximal 20 est, par exemple, également de forme cylindrique s'étendant selon l'axe A. Il présente un diamètre supérieur au diamètre de l'extrémité distale 18 et avantageusement un diamètre inférieur à la dimension transversale de la surface interne 32 cylindrique de la collerette.

L'épaulement 70 est situé entre l'extrémité distale 18 et le corps proximal 20. Il est sensiblement perpendiculaire à l'axe A.

L'extrémité distale 18 de la seringue 16 illustrée sur la figure 6 est propre à se monter dans l'embout 10, de sorte que la partie distale 66 tronconique soit en contact avec la surface interne 34 tronconique de la partie intermédiaire 28 suivant une surface de contact annulaire 57 au niveau de l'interface entre la partie distale 66 et la partie proximale 68 de l'extrémité distale 18. Lorsque l'embout 10 est monté sur la seringue 16, l'épaulement 70 de la seringue 16 repose par exemple sur l'épaulement 40 de la collerette 26 de l'embout 10.

Avantageusement, la dimension transversale minimale de l'extrémité distale 18 de la seringue 16 contenue dans le kit de filtration est comprise entre 2 et 10 mm.

La contenance de la seringue 16 du kit de filtration est par exemple comprise entre 1 mL et 5 mL.

Le kit de filtration peut également contenir une compresse stérile, et/ou du produit désinfectant, et/ou un manuel d'utilisation de l'embout, et/ou tout autre élément adapté.

Un procédé d'utilisation du kit de filtration va maintenant être décrit.

Tout d'abord, le produit injectable est fourni, par exemple sous forme liquide dans un récipient.

L'utilisateur saisit ensuite une seringue 16 présente dans le kit de filtration.

La partie intermédiaire 28 de l'embout 10 est ensuite montée sur l'extrémité distale 18 de la seringue 16 saisie. L'extrémité distale 18 est ainsi en contact étanche avec la surface interne 34 tronconique de la partie intermédiaire 28 suivant une surface de contact annulaire 57.

Une dose du produit injectable est ensuite aspirée à travers les surfaces de filtration 54 de la grille de maintien 50, et est ensuite filtrée par la membrane filtrante 14. La dose du produit injectable filtrée est finalement aspirée dans la lumière 24 axiale jusqu'à la seringue 16.

L'embout 10 est ensuite retiré de la seringue 16, puis jeté.

L'utilisateur peut ensuite s'injecter le produit injectable, dont les impuretés ont été filtrées, à l'aide de la seringue 16.

L'embout de filtration 10 décrit offre l'avantage d'être compatible avec des seringues de formes et de diamètres variés.

La forme tronconique de la surface interne 34 de la partie intermédiaire 28 présente l'avantage de pouvoir être monté sur des extrémités distales 18 de seringues de diamètres très variés. En effet, plus le diamètre de l'embout distale 18 de la seringue 16 est petit, plus l'anneau de contact 57 sera localisé proche de la tête plate 30 d'aspiration.

Cette compatibilité de l'embout de filtration 10 avec un grand nombre de seringues évite une étape de modification de l'embout afin de l'adapter à la seringue ou de devoir utiliser un adaptateur. Ce qui présente un avantage économique.

Il est ainsi possible de former un premier kit de filtration avec un premier type de seringue 16 et un deuxième kit de filtration avec un deuxième type de seringue 16.

Les seringues 16 du premier kit de filtration et du deuxième kit de filtration présentent des extrémités distales 18 de forme et/ou de dimension transversale différentes.

Par exemple, la seringue 16 du premier kit de filtration comprend une extrémité distale 18 de forme cylindrique raccordée à un corps proximal 20 de la seringue 16 par un épaulement 56 perpendiculaire à l'axe de la seringue 16. Alors que la seringue 16 du deuxième kit de filtration comprend une jupe externe 62 autour de l'extrémité distale 18.

Avantageusement, étant donné qu'un embout de filtration 10 est propre à recevoir des seringues 16 de structures très différentes, les embouts de filtration 10 du premier kit de filtration et du deuxième kit de filtration sont identiques. On forme ainsi un ensemble de kits de filtrations avec des embouts 10 standardisés et des seringues différentes.

Par ailleurs, l'embout 10 de filtration est simple d'utilisation.

La mise en contact de l'extrémité distale 18 de la seringue 16 avec la surface interne 34 tronconique de la partie intermédiaire 28 garantit l'étanchéité du montage de l'embout 10 sur la seringue 16.

De plus, les zones de maintien 56 de la membrane filtrante 14 évitent que la membrane 14 ne se perce en cas d'aspiration trop prononcée et donc garantit une filtration sûre de l'intégralité de la dose de produit injectable.

## Revendications

1. Embout de filtration (10) d'un produit injectable destiné à être monté à l'extrémité distale (18) d'une seringue (16) comprenant :
- un corps d'embout (12) définissant une lumière axiale (24) de passage du produit injectable comprenant :
+ une collerette (26) d'extrémité proximale, la collerette (26) définissant une surface interne cylindrique (32) ;
+ une partie intermédiaire (28) destinée à recevoir une extrémité distale (18) de la seringue (16) ;
+ une tête plate d'aspiration (30) du produit injectable d'extrémité distale, définissant une ouverture d'aspiration (42) du produit injectable de dimension transversale supérieure à la dimension transversale minimale de la lumière axiale (24) de passage ; et
- une membrane filtrante (14) montée dans l'ouverture d'aspiration (42) de la tête d'aspiration (30);
**caractérisé en ce que** la partie intermédiaire (28) définit une surface interne tronconique (34) s'étendant continument depuis la collerette (26) jusqu'à la tête plate d'aspiration (30); la surface interne tronconique (34) de la partie intermédiaire (28) délimitant la périphérie de la lumière axiale (24) de passage.

2. Embout de filtration selon la revendication 1, dans lequel la surface interne tronconique (34) de la partie intermédiaire (28) s'étend de manière continument dérivable depuis la collerette (26) jusqu'à la tête plate d'aspiration (30).

3. Embout de filtration selon la revendication 1 ou 2, dans lequel l'angle au sommet de la surface interne tronconique (34) de la partie intermédiaire (28) est compris entre 3° et 10°.

4. Embout de filtration selon l'une quelconque des revendications précédentes, dans lequel la dimension transversale de la surface interne tronconique (34) au niveau de l'interface (36) entre la collerette (26) et la partie intermédiaire (28) est comprise entre 2 mm et 10 mm.

5. Embout de filtration selon l'une quelconque des revendications précédentes, dans lequel le corps d'embout (12) est en matière plastique.

6. Embout de filtration selon l'une quelconque des revendications précédentes comprenant une grille de maintien (50) de la membrane filtrante (14) située distalement par rapport à la membrane filtrante (14).

7. Embout de filtration selon la revendication 6, dans lequel la grille de maintien (50) comprend des passages définissant des surfaces de filtration (52) du produit injectable.

8. Embout de filtration selon la revendication 6 ou 7, dans lequel la tête plate d'aspiration (30) comprend des butées (46) ; chaque butée (46) définit une zone de maintien (52) de la membrane filtrante (14) contre la grille de maintien (50).

9. Kit de filtration d'un produit injectable comprenant un embout de filtration (10) selon l'une quelconque des revendications précédentes et au moins une seringue (16).

10. Kit de filtration selon la revendication 9, dans lequel la seringue (16) comprend :
- une extrémité distale (18) de forme cylindrique raccordée à un corps proximal (20) de la seringue (16) par un épaulement perpendiculaire (56) ;
- une extrémité distale tronconique (18) raccordée à un corps proximal (20) de la seringue (16) par un épaulement de forme arrondie concave (58) ;
- une extrémité distale (18) et une jupe externe (62) entourant l'extrémité distale (18), un espace annulaire (64) étant défini entre la jupe externe (62) et l'extrémité distale (18) de la seringue (16) ; ou
- une extrémité distale (18) comprenant une partie distale tronconique (66) et une partie proximale cylindrique (68) raccordée à un corps proximal (20) de la seringue (16) par un épaulement perpendiculaire (70).

11. Kit de filtration selon la revendication 9 ou 10, dans lequel la dimension transversale de l'extrémité distale (18) de la seringue (16) est comprise entre 2 et 10 mm.

12. Ensemble de kits de filtration comprenant un premier kit de filtration selon l'une quelconque des revendications 9 à 11 et un deuxième kit de filtration selon l'une quelconque des revendications 9 à 11 ; les embouts de filtration (10) du premier kit de filtration et du deuxième kit de filtration étant identiques et les seringues (16) du premier kit de filtration et du deuxième kit de filtration présentant des extrémités distales (18) de forme et/ou de dimension transversale différentes.

## Patentansprüche

1. Filtrationsendstück (10) für ein injizierbares Produkt, das dazu bestimmt ist, an dem distalen Ende (18) einer Spritze (16) montiert zu werden, umfassend:
- einen Endstückkörper (12), der ein axiales Durchgangslumen (24) des injizierbaren Produkts definiert, umfassend:
+ einen proximalen Endflansch (26), wobei der Flansch (26) eine zylindrische Innenfläche (32) definiert;
+ ein Zwischenabschnitt (28), der dazu bestimmt ist, ein distales Ende (18) der Spritze (16) aufzunehmen;
+ einen flachen Ansaugkopf (30) für das injizierbare Produkt mit distalem Ende, der eine Ansaugöffnung (42) für das injizierbare Produkt definiert, deren Querabmessung größer ist als die minimale Querabmessung des axialen Durchgangslumens (24); und
- eine Filtermembran (14), die in der Ansaugöffnung (42) des Ansaugkopfs (30) montiert ist;
**dadurch gekennzeichnet, dass** der Zwischenabschnitt (28) eine kegelstumpfförmige Innenfläche (34) definiert, die sich kontinuierlich von dem Flansch (26) zu dem flachen Saugkopf (30) erstreckt; wobei die kegelstumpfförmige Innenfläche (34) des Zwischenabschnitts (28) den Umfang des axialen Durchgangslumens (24) begrenzt.

2. Filtrationsendstück nach Anspruch 1, wobei sich die kegelstumpfförmige Innenfläche (34) des Zwischenabschnitts (28) kontinuierlich ableitbar von dem Flansch (26) zu dem flachen Ansaugkopf (30) erstreckt.

3. Filtrationsendstück nach Anspruch 1 oder 2, wobei der Winkel an der Spitze der kegelstumpfförmigen Innenfläche (34) des Zwischenabschnitts (28) zwischen 3° und 10° ist.

4. Filtrationsendstück nach einem der vorherigen Ansprüche, wobei die Querabmessung der kegelstumpfförmigen Innenfläche (34) an der Schnittstelle (36) zwischen dem Flansch (26) und dem Zwischenabschnitt (28) zwischen 2 mm und 10 mm ist.

5. Filtrationsendstück nach einem der vorherigen Ansprüche, wobei der Endstückkörper (12) aus Kunststoff ist.

6. Filtrationsendstück nach einem der vorherigen Ansprüche, umfassend ein Haltegitter (50) für die Filtermembran (14), das sich distal von der Filtermembran (14) befindet.

7. Filtrationsendstück nach Anspruch 6, wobei das Haltegitter (50) Durchgänge umfasst, die Filtrationsflächen (52) für das injizierbare Produkt definieren.

8. Filtrationsendstück nach Anspruch 6 oder 7, wobei der flache Ansaugkopf (30) Anschläge (46) umfasst; jeder Anschlag (46) einen Bereich (52) definiert, in dem die Filtermembran (14) an dem Haltegitter (50) gehalten wird.

9. Filtrationskit eines injizierbaren Produkts, umfassend ein Filtrationsendstück (10) nach einem der vorherigen Ansprüche und mindestens eine Spritze (16).

10. Filtrationskit nach Anspruch 9, wobei die Spritze (16) Folgendes umfasst:
- ein zylinderförmiges distales Ende (18), das über eine senkrechte Schulter (56) mit einem proximalen Körper (20) der Spritze (16) verbunden ist;
- ein kegelstumpfförmiges distales Ende (18), das über eine konkave, rund geformte Schulter (58) mit einem proximalen Körper (20) der Spritze (16) verbunden ist;
- ein distales Ende (18) und eine äußere Schürze (62), die das distale Ende (18) umgibt, wobei zwischen der äußeren Schürze (62) und dem distalen Ende (18) der Spritze (16) ein ringförmiger Raum (64) definiert ist; oder
- ein distales Ende (18), das einen kegelstumpfförmigen distalen Abschnitt (66) und einen zylindrischen proximalen Abschnitt (68) umfasst, der über eine senkrechte Schulter (70) mit einem proximalen Körper (20) der Spritze (16) verbunden ist.

11. Filtrationskit nach Anspruch 9 oder 10, wobei die Querabmessung des distalen Endes (18) der Spritze (16) zwischen 2 und 10 mm ist.

12. Anordnung von Filtrationskits, umfassend ein erstes Filtrationskit nach einem der Ansprüche 9 bis 11 und ein zweites Filtrationskit nach einem der Ansprüche 9 bis 11; wobei die Filtrationsendstücke (10) des ersten Filtrationskits und des zweiten Filtrationskits identisch sind und die Spritzen (16) des ersten Filtrationskits und des zweiten Filtrationskits distale Enden (18) mit unterschiedlicher Form und/oder Querschnittsabmessung aufweisen.

## Claims

1. Filtration tip (10) for an injectable product intended to be mounted at the distal end (18) of a syringe (16), comprising:
- a tip body (12) defining an axial slot (24) for passage of the injectable product comprising:
+ a flange (26) at the proximal end, said flange (26) defining a cylindrical inner surface (32);
+ an intermediate part (28) intended to receive a distal end (18) of the syringe (16);
+ a flat suction head (30) for the injectable product at the distal end, defining a suction opening (42) for the injectable product of transverse dimension greater than the minimum transverse dimension of the axial slot (24); and
- a filter membrane (14) mounted in the suction opening (42) of the suction head (30);
**characterised in that** the intermediate part (28) defines a frustoconical inner surface (34) extending continuously from the flange (26) to the flat suction head (30); the frustoconical inner surface (34) of the intermediate part (28) delimiting the periphery of the axial slot (24).

2. Filtration tip according to claim 1, in which the frustoconical inner surface (34) of the intermediate part (28) extends in a continuously derivable manner from the flange (26) to the flat suction head (30).

3. Filtration tip according to claim 1 or 2, in which the angle at the apex of the frustoconical inner surface (34) of the intermediate part (28) is between 3° and 10°.

4. Filtration tip according to any one of the preceding claims, in which the transverse dimension of the frustoconical inner surface (34) at the interface (36) between the flange (26) and the intermediate part (28) is between 2 mm and 10 mm.

5. Filtration tip according to any one of the preceding claims, in which the tip body (12) is made of plastic.

6. Filtration tip according to any one of the preceding claims, comprising a holding grid (50) for the filter membrane (14), located distally with respect to the filter membrane (14).

7. Filtration tip according to claim 6, in which the holding grid (50) comprises passages defining filtration surfaces (54) for the injectable product.

8. Filtration tip according to claim 6 or 7, wherein the flat suction head (30) comprises stops (46); each stop (46) defines a holding zone (52) holding the filter membrane (14) against the holding grid (50).

9. Filtration kit for an injectable product, comprising a filtration tip (10) according to any one of the preceding claims and at least one syringe (16).

10. Filtration kit according to claim 9, wherein the syringe (16) comprises:
- a distal end (18) of cylindrical shape connected to a proximal body (20) of the syringe (16) by a perpendicular shoulder (56);
- a frustoconical distal end (18) connected to a proximal body (20) of the syringe (16) by a concave rounded shoulder (58);
- a distal end (18) and an outer skirt (62) surrounding the distal end (18), an annular space (64) being defined between the outer skirt (62) and the distal end (18) of the syringe (16); or
- a distal end (18) comprising a frustoconical distal part (66) and a cylindrical proximal part (68) connected to a proximal body (20) of the syringe (16) by a perpendicular shoulder (70).

11. Filtration kit according to claim 9 or 10, wherein the transverse dimension of the distal end (18) of the syringe (16) is between 2 mm and 10 mm.

12. Set of filtration kits comprising a first filtration kit according to any one of claims 9 to 11 and a second filtration kit according to any one of claims 9 to 11; the filtration tips (10) of the first filtration kit and the second filtration kit being identical, and the syringes (16) of the first filtration kit and the second filtration kit having distal ends (18) that differ in shape and/or transverse dimension.
